# EUROPÄISCHE PATENTANMELDUNG

(11) **EP 0 529 400 A1**
(43) Veröffentlichungstag der Anmeldung: **03.03.1993**
(21) Anmeldenummer: 92113667.7
(22) Anmeldetag: 11.08.1992
(51) Int. Cl.: C07D 207/34, C07C 255/23, A01N 43/36

(54) **3-(2-Chlor-3-trifluormethylphenyl)-4-cyanopyrrol, als Schädlungsbekämpfungsmittel**

(30) Priorität: 24.08.1991 DE 4128132
(71) Anmelder: BAYER AG, 51368 Leverkusen (DE)
(72) Erfinder: Heinemann, Ulrich, Dr., W-5653 Leichlingen 2 (DE); Marhold, Albrecht, Dr., W-5090 Leverkusen (DE); Dutzmann, Stefan, Dr., W-4010 Hilden (DE); Dehne, Heinz-Wilhelm, Dr. habil., W-4019 Monheim (DE)

(57) **Zusammenfassung**

Das neue 3-(2-Chlor-3-trifluormethylphenyl)-4-cyanopyrrol (I) und seine Verwendung zur Bekämfpung von Schädlingen.

Die Verbindung kann nach Analogieverfahren hergestellt werden, z. B. aus 2-Chlor-3-Trifluormethylbenzaldehyd und einem geeignetem Cyanessigsäurealkylester und p-Toluolsulfonyl-methyl-isocyanid. Die als Zwischenprodukte auftretenden α-Cyano-zimtsäureester (II) sind ebenfalls neu und Gegenstand der Erfindung.

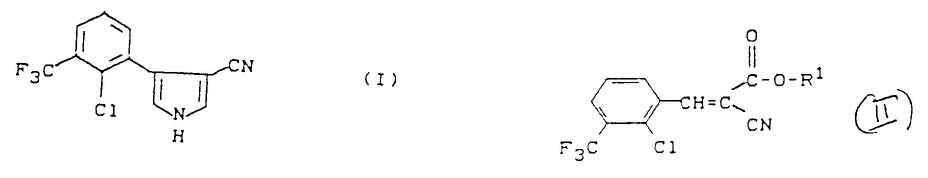

## Beschreibung

Die Erfindung betrifft die neue Verbindung 3-(2-Chlor-3-trifluormethylphenyl)-4-cyanopyrrol, ein Verfahren zu ihrer Herstellung und ihre Verwendung als Schädlingsbekämpfungsmittel und neue Zwischenprodukte.

Es ist bekannt, daß bestimmte 3-Aryl-4-cyanopyrrol-Verbindungen, wie beispielsweise die Verbindung 3-(3-Trifluormethylphenyl)-4-cyanopyrrol fungizide Eigenschaften besitzen (vgl. z.B. EP 96 142).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurde die neue Verbindung 3-(2-Chlor-3-trifluormethylphenyl)-4-cyanopyrrol der Formel (I) 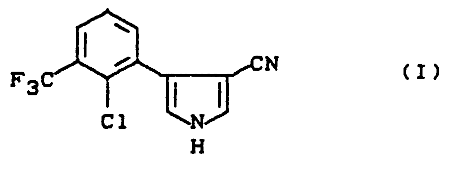
gefunden.

Weiterhin wurde gefunden, daß man die neue Verbindung 3-(2-Chlor-3-trifluormethylphenyl)-4-cyanopyrrol der Formel (I) 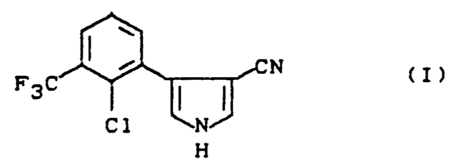
erhält, wenn man 2-Chlor-3-trifluormethylbenzaldehyd der Formel (II) 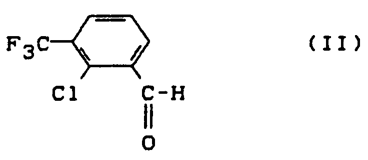
zunächst in einer ersten Stufe mit Cyanessigsäurealkylestern der Formel (III), 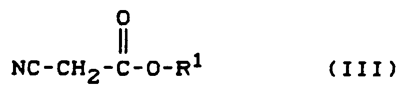
in welcher
- R¹: für Alkyl steht,
gegebenenfalls in Gegenwart eines Verdünnungsmittels und gegebenenfalls in Gegenwart eines Reaktionshilfsmittels umsetzt und anschließend die so erhältlichen α-Cyanozimtsäureester der Formel (IV), 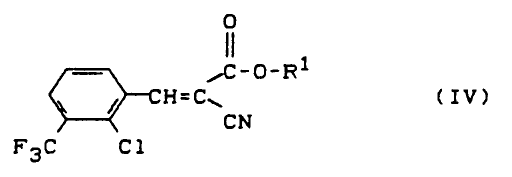
in welcher
- R¹: die oben angegebene Bedeutung hat,
mit p-Toluolsulfonylmethylisocyanid (TOSMIC) der Formel (V) 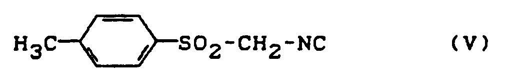
gegebenenfalls in Gegenwart eines Verdünnungsmittels und gegebenenfalls in Gegenwart eines Reaktionshilfsmittels umsetzt.

Schließlich wurde gefunden, daß die neue Verbindung 3-(2-Chlor-3-trifluormethylphenyl)-4-cyanopyrrol der Formel (I) gute Wirksamkeit gegenüber Schädlingen besitzt.

Überraschenderweise zeigt die neue Verbindung 3-(2-Chlor-3-trifluormethylphenyl)-4-cyanopyrrol der Formel (I) eine erheblich bessere fungizide Wirksamkeit gegenüber pflanzenschädigenden Pilzen als die aus dem Stand der Technik bekannten 3-Aryl-4-cyanopyrrol-Verbindungen, wie beispielsweise die Verbindung 3-(3-Trifluormethylphenyl)-4-cyanopyrrol, welches chemisch und wirkungsmäßig naheliegende Verbindungen sind.

Verwendet man beispielsweise 2-Chlor-3-trifluormethylbenzaldehyd und Cyanessigsäureethylester als Ausgangsverbindungen, so läßt sich der Reaktionsablauf des erfindungsgemäßen Verfahrens durch das folgende Formelschema darstellen:

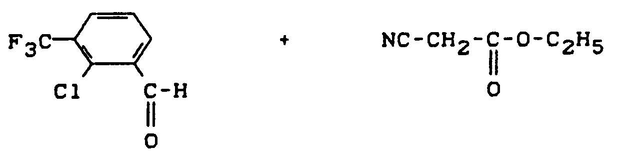

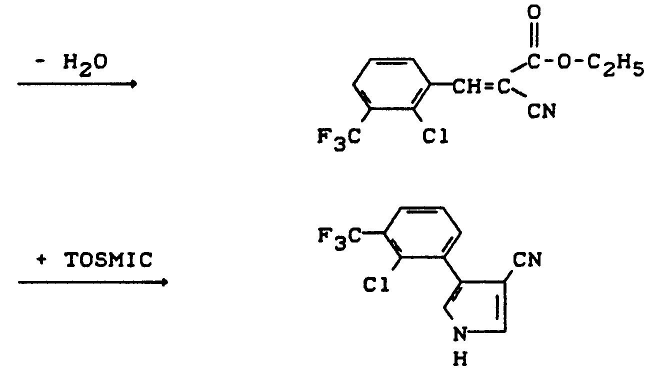

Der zur Durchführung des erfindungsgemäßen Verfahrens als Ausgangsverbindung benötigte 2-Chlor-3-trifluormethylbenzaldehyd ist durch die Formel (II) definiert. Der 2-Chlor-3-trifluormethylbenzaldehyd der Formel (II) ist bekannt (vgl. z.B. US 4.572.909; EP 145 334; JP 59118782).

Die zur Durchführung des erfindungsgemäßen Verfahrens weiterhin als Ausgangsstoffe benötigten Cyanessigsäurealkylester sind durch die Formel (III) allgemein definiert. In dieser Formel (III) steht R¹ vorzugsweise für geradkettiges oder verzweigtes Alkyl mit 1 bis 6, insbesondere 1 bis 4 Kohlenstoffatomen. Die Cyanessigsäurealkylester der Formel (III) sind allgemein bekannte Verbindungen der organischen Chemie.

Das zur Durchführung des erfindungsgemäßen Verfahrens weiterhin als Ausgangsstoff benötigte p-Toluolsulfonylmethylisocyanid (TOSMIC) ist durch die Formel (V) allgemein definiert. Das p-Toluolsulfonylmethylisocyanid (TOSMIC) der Formel (V) ist bekannt (vgl. z.B. Synthesis 1985, 400-402; J. Org. Chem. 42, 1153-1159 [1977]; Tetrahedron Lett. 1972, 2367-2368).

Die bei der Durchführung des erfindungsgemäßen Verfahrens als Zwischenprodukte auftretenden α-Cyano-zimtsäureester der Formel (IV) sind noch nicht bekannt und ebenfalls Gegenstand der Erfindung.

Als Verdünnungsmittel zur Durchführung der ersten und zweiten Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Die 1. Stufe des erfindungsgemäßen Verfahrens wird vorzugsweise in Gegenwart eines geeigneten Reaktionshilfsmittels durchgeführt. Als solche kommen alle üblichen anorganischen oder organischen Säuren oder Basen in Frage. Hierzu gehören beispielsweise Erdalkali- oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat oder Ammoniumcarbonat, Alkali-oder Erdalkalimetallacetate wie Natriumacetat, Kaliumacetat, Calciumacetat oder Ammoniumacetat sowie tertiäre Amine wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU) oder auch Säuren, wie beispielsweise p-Toluolsulfonsäure.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 0°C und 160°C, vorzugsweise bei Temperaturen zwischen 20°C und 120°C.

Die erste Stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der ersten Stufe des erfindungsgemäßen Verfahrens setzt man pro Mol an 2-Chlor-3-trifluormethylbenzaldehyd der Formel (II) im allgemeinen 1.0 bis 5,0 Mol, vorzugsweise 1.0 bis 1,5 Mol an Cyanessigsäurealkylester der Formel (III) und gegebenenfalls 1.0 bis 5,0 Mol, vorzugsweise 1.0 bis 2,5 Mol an Reaktionshilfsmittel ein.
Die Reaktionsdurchführung, Aufarbeitung und Isolierung der Reaktionsprodukte erfolgt nach allgemein üblichen Methoden (vgl. hierzu beispielsweise JP 58116462 oder die Herstellungsbeispiele).

Die zweite Stufe des erfindungsgemäßen Verfahrens wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen -30°C und +40°C, vorzugsweise bei Temperaturen zwischen -20°C und +20°C.

Die zweite Stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der zweiten Stufe des erfindungsgemäßen Verfahrens setzt man pro Mol an α-Cyano-zimtsäureester der Formel (IV) im allgemeinen 1.0 bis 2,0 Mol, vorzugsweise 1.0 bis 1,2 Mol an p-Toluolsulfonylmethylisocyanid (TOSMIC) der Formel (V) und gegebenenfalls 1.0 bis 2,0 Mol, vorzugsweise 1.0 bis 1,2 Mol an als Reaktionshilfsmittel verwendeter Base ein. Die Reaktionsdurchführung, Aufarbeitung und Isolierung der Reaktionsprodukte erfolgt nach allgemein üblichen Methoden (vgl. hierzu beispielsweise JP 58116462 oder die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum; Phytophthora-Arten, wie beispielsweise Phytophthora infestans; Pseudoperonospora-Arten, wie beispielsweise Pseudoperonospora humuli oder Pseudoperonospora cubensis; Plasmopara-Arten, wie beispielsweise Plasmopara viticola; Peronospora-Arten, wie beispielsweise Peronospora pisi oder Peronospora brassicae; Erysiphe-Arten, wie beispielsweise Erysiphe graminis; Sphaerotheca-Arten, wie beispielsweise Sphaerotheca fuliginea; Podosphaera-Arten, wie beispielsweise Podosphaera leucotricha; Venturia-Arten, wie beispielsweise Venturia inaequalis; Pyrenophora-Arten, wie beispielsweise Pyrenophora teres oder Pyrenophora graminea (Konidienform: Drechslera, Synonym: Helminthosporium); Cochliobolus-Arten, wie beispielsweise Cochliobolus sativus (Konidienform: Drechslera, Synonym: Helminthosporium); Uromyces-Arten, wie beispielsweise Uromyces appendiculatus; Puccinia-Arten, wie beispielsweise Puccinia recondita; Tilletia-Arten, wie beispielsweise Tilletia caries; Ustilago-Arten, wie beispielsweise Ustilago nuda oder Ustilago avenae; Pellicularia-Arten, wie beispielsweise Pellicularia sasakii; Pyricularia-Arten, wie beispielsweise Pyricularia oryzae; Fusarium-Arten, wie beispielsweise Fusarium culmorum; Botrytis-Arten, wie beispielsweise Botrytis cinerea; Septoria-Arten, wie beispielsweise Septoria nodorum; Leptosphaeria-Arten, wie beispielsweise Leptosphaeria nodorum; Cercospora-Arten, wie beispielsweise Cercospora canescens; Alternaria-Arten, wie beispielsweise Alternaria brassicae; Pseudocercosporella-Arten, wie beispielsweise Pseudocercosporella herpotrichoides. Die gute Pflanzenverträglichkeit der Wirkstoffe in den zur Bekämpfung von Pflanzenkrankheiten notwendigen Konzentrationen erlaubt eine Behandlung von oberiridischen Pflanzenteilen, von Pflanz- und Saatgut und des Bodens.

Dabei können die erfindungsgemäßen Wirkstoffe mit besonders gutem Erfolg zur Bekämpfung von Getreidekrankheiten, wie beispielsweise gegen den Erreger des echten Getreidemehltaues (Erysiphe graminis) oder gegen den Erreger des Getreideschneeschimmels (Fusarium nivale) oder zur Bekämpfung von Krankheiten im Obst- und Gemüseanbau, wie beispielsweise gegen den Erreger des Grauschimmels an Bohnen (Botrytis cinerea) oder zur Bekämpfung von Reiskrankheiten, wie beispielsweise gegen den Erreger der Reisfleckenkrankheit (Pyricularia oryzae) eingesetzt werden. Daneben besitzen die erfindungsgemäßen Wirkstoffe eine breite in vitro-Wirksamkeit. Darüberhinaus zeigen die erfindungsgemäßen Wirkstoffe in entsprechenden Aufwandmengen auch eine blattinsektizide Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.
Es können Farbstoffe wie anorganische Pigmente, z.B. Eisenoxid, Titanoxid, Ferrocyanblau und organische Farbstoffe, wie Alizarin-, azo- und Metallphthalocyaninfarbstoffe und Spurennähhrstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die erfindungsgemäßen Wirkstoffe zeichnen sich neben der oben aufgeführten Wirksamkeit gegen pflanzenpathogene Mikroorganismen durch eine breite und starke mikrobizide Wirkung gegen ein breites Spektrum von für den Materialschutz relevanten Mikroorganismen aus sowie durch auffallend gute Wirksamkeit gegen Algen und Schleimorganismen. Die erfindungsgemäßen Substanzen eignen sich daher vorzüglich zum Schutz technischer Materialien.

Technische Materialien in diesem Sinne sind nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunstoffartikel, Kühlschmierstoffe und andere Materialien sein, die von Mikroorganismen befallen oder zersetzt werden können. Im Rahmen der zu schützenden Materialien seien auch Teile von Produktionsanlagen, wie beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartons, Leder, Holz, Anstrichmittel, Kunststoffartikel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger;
Chaetomium, wie Chaetominum globosum;
Coniophora, wie Coniophora puteana;
Lentinus, wie Lentinus tigrinus;
Penicillium,wie Penicillium glaucum;
Polyporus, wie Polyporus versicolor;
Aureobasidium, wie Aureobasidium pullulans;
Sclerophoma, wie Sclerophoma pityophila;
Trichoderma, wie Trichoderma viride;
Escherichia, wie Escherichia coli;
Pseudomonas, wie Pseudomonas aeruginosa;
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten oder Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Fall der Benutzung von Wasser als Streckmittel organische Lösungsmittel wie Alkohole als Hilfsmittel verwendet werden können. Flüssige Lösungsmittel für die Wirkstoffe können beispielsweise Wasser, Alkohole, vorzugsweise Ethanol oder Isopropanol oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen errmittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(oder -poly)hemiformal und andere Formaldehyd abspaltende Verbindungen, Benzimidazolyl-methylcarbamate, Tetramethyldiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, Phenolderivate, wie 2-Phenylphenol, (2,2'-Dihydroxy-5,5'-dichlor)-diphenylmethan, 3-Methyl-4-chlorphenol, 2-Thiocyanatomethylthiobenzthiazol, N-Trihalogenmethylthioverbindungen, wie Folpet, Fluorfolpet und Dichlofluanid, Azolfungizide, wie Triadimefon, Triadimenol, Bitertanol, Tebuconazol, Propiconazol, Azoconazol, Jodpropargylderivate, wie Jodpropargylbutylcarbamat und Jodpropargylphenylcarbamat, Isothiazolinonverbindungen, wie Kathon sowie quartäre Ammoniumverbindungen, wie Benzalkoniumchlorid.
Ebenfalls können Mischungen der erfindungsgemäß zu verwendenden Substanzen mit bekannten Insektiziden Anwendung finden. Beispielhaft seien hier genannt: Organophosphorverbindungen, wie Chlorpyriphos oder Phoxim, Carbamate, wie Aldicarb, Carbosulfan oder Propoxur oder Pyrethroide, wie Permethrin, Cyfluthrin, Cypermethrin, Deltamethrin oder Fenvalerat.
Ebenso kommen als Mischpartner Algizide, Molluskizide sowie Wirkstoffe gegen "sea animals", die sich auf Schiffsbodenanstrichen ansiedeln in Frage.

### Herstellungsbeispiele:

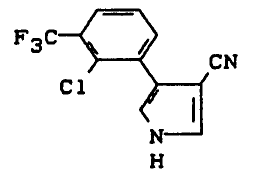

Zu 34,8 g (0,115 Mol) 2-Cyano-3-(2-chlor-3-trifluormethylphenyl)-acrylsäureethylester in 80 ml trockenem Ethanol gibt man bei 0°C bis 5°C tropfenweise unter Rühren eine Lösung von 3,5 g (0,154 Mol) Natrium in 80 ml Ethanol und anschließend ebenfalls bei 0°C bis 5°C tropfenweise unter Rühren eine Lösung von 23,6 g (0,121 Mol) p-Toluolsulfonylmethylisocyanid (TOSMIC) in 120 ml trokkenem Dichlormethan. Nach beendeter Zugabe rührt man eine weitere Stunde bei 0°C und 16 Stunden bei Raumtemperatur, stellt dann mit 2N Salzsäure den pH-Wert der Reaktionsmischung auf pH 8 ein, trennt die organische Phase ab, trocknet sie über Natriumsulfat, engt im Vakuum ein, nimmt den Rückstand in Dichlormethan auf, wäscht mit Wasser, trocknet über Natriumsulfat, engt im Vakuum ein und entfernt flüchtige Bestandteile im Hochvakuum. Der kristalline Rückstand wird durch Abpressen auf Ton getrocknet.

Man erhält 26,3 g (85% der Theorie) an 3-(2-Chlor-3-trifluormethylphenyl)-4-cyanopyrrol vom Schmelzpunkt 82°C.

### Herstellung der Ausgangsverbindung:

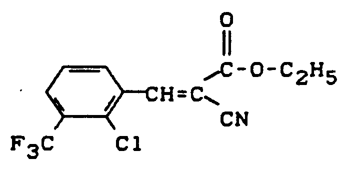

Zu 31,0 g (0,149 Mol) 2-Chlor-3-trifluormethylbenzaldehyd (vgl. z.B. EP 145 334) und 18,5 g (0,164 Mol) Cyanessigsäureethylester in 75 ml Toluol gibt man tropfenweise unter Rühren eine Lösung von 1 ml Piperidin in 5 ml Toluol, wobei die Temperatur der Reaktionsmischung auf ca. 40°C ansteigt. Nach beendeter Zugabe rührt man 2 Stunden bei 70°C, kühlt dann ab auf Raumtemperatur, wäscht dreimal mit Wasser, trocknet über Natriumsulfat und entfernt das Lösungsmittel im Vakuum.

Man erhält 37,3 g (83% der Theorie) an 2-Cyano-3-(2-chlor-3-trifluormethylphenyl)-acrylsäureethylester vom Schmelzpunkt 50°C.

### Anwendungsbeispiele:

In den folgenden Anwendungsbeispielen wurde die nachstehend aufgeführte Verbindung als Vergleichssubstanz eingesetzt: 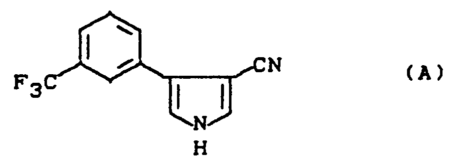
3-(3-Trifluormethylphenyl)-4-cyanopyrrol
(bekannt aus EP 96 142).

### Beispiel A;

### Erysiphe-Test (Weizen) / protektiv

- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca- 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test die erfindungsgemäße Verbindung.

### Beispiel B:

### Fusarium nivale-Test (Roggen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10°C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Eine deutliche Überlegenheit in der Wirksamkeit gegenüber dem Stand der Technik zeigt bei diesem Test die erfindungsgemäße Verbindung.

### Beispiel C:

### Botrytis-Test (Buschbohne)/protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

3 Tage nach der Inokulation wird die Größe der Befallsflecken auf den Blättern ausgewertet.

Eine deutliche Überlegenheit in der Wirksamkeit gegenüber dem Stand der Technik zeigt bei diesem Test die erfindungsgemäße Verbindung.

## Patentansprüche

1. 3-(2-Chlor-3-trifluormethylphenyl)-4-cyanopyrrol der Formel (I)

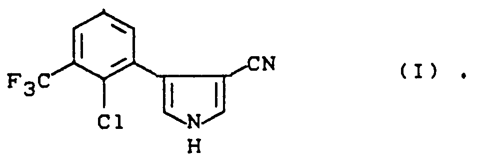

2. Verfahren zur Herstellung von 3-(2-Chlor-3-trifluormethylphenyl)-4-cyanopyrrol der Formel (I) 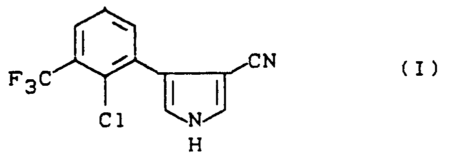 dadurch gekennzeichnet, daß man 2-Chlor-3-trifluormethylbenzaldehyd der Formel (II) 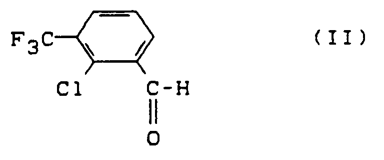 zunächst in einer ersten Stufe mit Cyanessigsäurealkylestern der Formel (III), 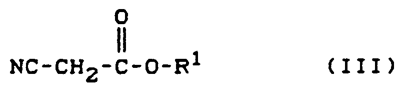 in welcher
R¹ für Alkyl steht,
gegebenenfalls in Gegenwart eines Verdünnungsmittels und gegebenenfalls in Gegenwart eines Reaktionshilfsmittels umsetzt und anschließend die so erhältlichen α-Cyano-zimtsäureester der Formel (IV), 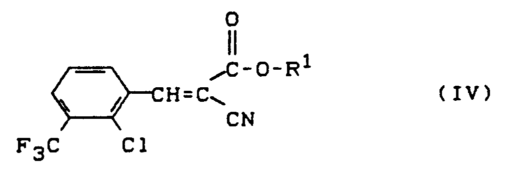 in welcher
R¹ die oben angegebene Bedeutung hat,
mit p-Toluolsulfonylmethylisocyanid (TOSMIC) der Formel (V) 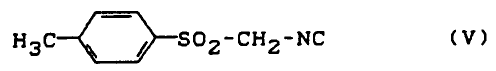 gegebenenfalls in Gegenwart eines Verdünnungsmittels und gegebenenfalls in Gegenwart eines Reaktionshilfsmittels umsetzt.

3. Schädlingsbekämpfungsmittel, gekennzeichnet durch einen Gehalt an 3-(2-Chlor-3-trifluormethylphenyl)-4-cyanopyrrol der Formel (I) nach den Ansprüchen 1 und 2.

4. Verwendung von 3-(2-Chlor-3-trifluormethylphenyl)-4-cyanopyrrol der Formel (I) nach den Ansprüchen 1 und 2 zur Bekämpfung von Schädlingen.

5. Verfahren zur Bekämpfung von Schädlingen, dadurch gekennzeichnet, daß man 3-(2-Chlor-3-trifluormethylphenyl-4-cyanopyrrol der Formel (I) nach den Ansprüchen 1 und 2 auf Schädlinge und/oder ihren Lebensraum einwirken läßt.

6. Verfahren zur Herstellung von Schädlingsbekämpfungsmitteln, dadurch gekennzeichnet, daß man 3-(2-Chlor-3-trifluormethylphenyl)-4-cyanopyrrol der Formel (I) nach den Ansprüchen 1 und 2 mit Streckmitteln und/oder oberflächenaktiven Mitteln vermischt.

7. α-Cyano-zimtsäureester der Formel (IV) 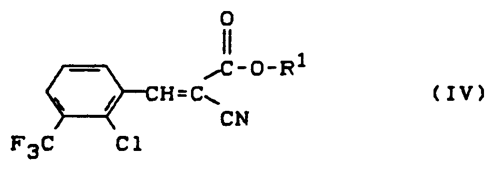 in welcher
R¹ für Alkyl steht.

8. Verfahren zur Herstellung von α-Cyanozimtsäureestern der Formel (IV) 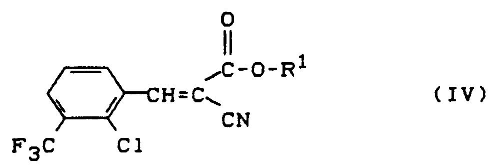 dadurch gekennzeichnet, daß man 2-Chlor-3-trifluormethylbenzaldehyd der Formel (II) 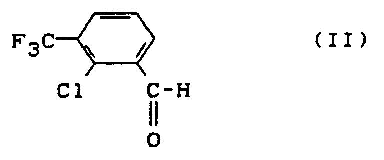 zunächst in einer ersten Stufe mit Cyanessigsäurealkylestern der Formel (III), 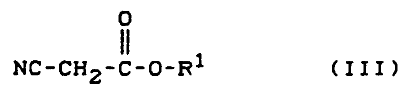 in welcher
R¹ für Alkyl steht,
gegebenenfalls in Gegenwart eines Verdünnungsmittels und gegebenenfalls in Gegenwart eines Reaktionshilfsmittels umsetzt.

9. Verwendung der α-Cyanozimtsäureester der Formel (IV) gemäß Ansprüchen 7 und 8 zur Herstellung von biologisch hochaktiven Verbindungen.
